# EUROPÄISCHE PATENTANMELDUNG

(11) **EP 1 632 171 A2**
(43) Veröffentlichungstag der Anmeldung: **08.03.2006**
(21) Anmeldenummer: 05104979.9
(22) Anmeldetag: 08.06.2005
(51) Int. Cl.: A61B 3/135, F21S 13/14, G02B 27/18

(54) **Beleuchtungsvorrichtung, insbesondere Spaltlampe**

(30) Priorität: 11.06.2004 DE 102004028471
(71) Anmelder: Leica Microsystems (Schweiz) AG, 9435 Heerbrugg (CH)
(72) Erfinder: Sander, Ulrich, 9445, Rebstein (CH)
(74) Vertreter: Reichert, Werner Franz

(57) **Zusammenfassung**

Die Erfindung betrifft eine Spaltlampe mit einem Leuchtmittel (2) und einer Freiformfläche (13) sowie einer Spaltblende (9) und einer Abbildungsoptik (10).

Der erfindungsgemäße Gedanke liegt darin, dass die Freiformfläche (13) zur Erzeugung eines rechteckigen Beleuchtungsstrahlengangs herangezogen wird.

Der Vorteil dieses Aufbaus besteht darin, dass die Lichtstrahlen nach dem Lichtaustritt aus der Linse (12) mit der Freiformfläche (13) spaltförmig in die Richtung des Patientenauges abgelenkt werden, ohne dass es zu nennenswertem Lichtverlust an der Spaltblende (9) kommt.

## Beschreibung

Die Erfindung betrifft eine Beleuchtungsvorrichtung, insbesondere eine Spaltlampe, die zur Operation und Diagnose bestimmter Augenerkrankungen eingesetzt wird.

Unter einer Spaltlampe versteht man ein Gerät, das zur Betrachtung des vorderen Augen-Abschnitts (Linse und naher Glaskörper) sowie zur Kontrolle bei Kontaktlinsen-Anpassungen verwendet wird. Die Spaltabbildung sorgt für die Erleichterung der Diagnose- bzw. Operationsvorgänge. Bei Operationen wird sie als kontraststeigernde Beleuchtung im Bereich der Retina eingesetzt.

Solche Spaltlampen sind bestens bekannt und werden von mehreren Anbietern hergestellt und von Ärzten routinemäßig verwendet.

Zum Stand der Technik gehören Spaltlampen, bei denen mittels eines Leuchtmittels eine Spattblende direkt oder über einen Lichtleiter beleuchtet wird.

In der DE-C2-42 27 942 ist beispielsweise eine Anordnung einer Spaltlampe mit einem Lichtleiter beschrieben:
"Spaltlampen-Beleuehtungseinrichtung zur Abbildung einer Spaltblende in eine Zielebene, bestehend aus
   - einer Lichtquelle, vorzugsweise hoher Intensität, der ein faseroptischer Lichtleiter nachgeordnet ist,
   - einer der Lichtleiter-Antrittsfläche in geringem Abstand ohne zwischengeschaltete optische Elemente nachgeordneten Spaltblende sowie
   - einer der Lichtleiter-Austrittsfläche in geringem Abstand ohne zwischengeschaltete optische Elemente nachgeordneten Spaltblende, sowie
   - einer der Spaltblende nachgeordneten Abbildungseinrichtung, mit mindestens zwei optischen Systemen mit jeweils sammeinder optischer Wirkung...".

Bei der oben genannten Ausführungsform ist eine Spaltblende direkt nach der Lichtleiter-Austrittsfläche angeordnet. Die Lichtleiter-Austrittsfläche verfügt jedoch über einen runden Querschnitt. Ein Teil des aus dem Lichtleiter austretenden Lichts beleuchtet den Spalt in der Ebene der Spaltblende. Dieser Spalt wird durch eine Abbildungsoptik auf das Patientenauge abgebildet. Ein wesentlich größerer Teil des Lichts wird jedoch an der mechanischen Spaltblende absorbiert und in Wärme umgewandelt und trägt somit nicht zur intensiven Ausleuchtung des Spaltbildes auf dem Patientenauge bei.

Aus dieser herkömmlichen Ausführungsform ergibt sich somit das Problem, dass durch den Übergang von der Kreisfläche (Austrittsfläche des Lichtleiters) zur Rechteckfläche (Eintrittsfläche der Spaltblende) erhebliche Anteile des nutzbaren Lichts aus dem Lichtleiter bzw. aus jedem anderen Leuchtmittel verloren gehen.

Dieses Problem ist nicht unbekannt, jedoch sind die Lösungsansätze, um die sich daraus ergebenden Mängel zu beseitigen, unzureichend. So wird z.B. zwischen der Austrittsfläche des Leuchtmittels und der Spaltblende eine Zylinderlinse eingebaut. Dadurch wird der Spalt nicht mehr kreisrund, sondern eher oval oder möglichst der Spaltform entsprechend beleuchtet. Aber einerseits verhindert diese Maßnahme den Lichtverlust nicht nennenswert und andererseits wird durch eine Zylinderlinse die Lichtverteilung im Spalt inhomogen.

Der Erfinder erkannte die Probleme der oben angegebenen Lösungen, die versuchen, den nutzbaren Anteil des Lichts zu erhöhen. Er versuchte, über den erfinderischen Gedanken eine möglichst effiziente Lichtausnutzung in dem Spaltbild unter gleichzeitiger Vermeidung der Absorption des nutzbaren Lichts auf der Spaltblende zu erreichen.

Der Erfindung liegt somit eine Aufgabe zugrunde, eine Spaltlampe zu schaffen, die die angegebenen Nachteile vermeidet.

Gelöst wird diese Aufgabe durch den Einsatz einer Freiformfläche (anisotrop gekrümmte Fläche), die nach dem Leuchtmittel (z.B. eine Halogenleuchte oder eine LED oder eine Lichtleiter-Austrittsfläche) angeordnet ist und über eine besondere Eigenschaft verfügt. Unter einer Freiformflächen-Linse ist im Sinne der Erfindung ein optisches Bauteil zu verstehen, das durch seine Ausgestaltung eine zielorientierte Lichtführung erlaubt. Insbesondere ist darunter eine Linse mit einer speziell gestalteten Oberflächentopographie zu verstehen, die es ermöglicht, dass auf sie auftreffende Lichtstrahlen in eine gewünschte Richtung weitergeleitet werden, die nicht der Richtung aus der Abbildung gemäß einer sphärischen Linse entspricht.

Die Freiformfläche ermöglicht nicht nur eine rotationssymmetrische Abweichung von der Sphäre als erste Näherung entsprechend einer Asphäre, sondern auch eine beliebige Änderung des KrümmungsRadius bezüglich jedes Flächenpunktes der Linsenoberfläche. Während bei bisherigen Abbildungssystemen die abbildenden Linsen runde Querschnitte des Strahlenbüschels des Beleuchtungs-Strahlengangs produzieren, wird durch entsprechende Linsen mit den neuartigen Freiformflächen der Büschelquerschnitt beliebig gestaltbar. So ist es z.B. möglich, einen runden Büschelquerschnitt in einen rechteckigen umzuwandeln. Die Umwandlung in den gewünschten Querschnitt geschieht nicht unmittelbar an der Austrittsfläche der Freiformflächen-Linse; das Lichtbüschel formt sich erst nach dem Austritt aus der Linse.

Vorzugsweise ist das optische Element mit der Freiformfläche eine Linse, es kann jedoch auch eine Platte oder ein Prisma sein. Allen diesen optischen Elementen ist erfindungsgemäß gemeinsam, dass sie die Gesamtheit des runden Querschnitts des Strahlenbüschels aufnehmen. Dieses geschieht bei einer Linse oder einer Platte auf platzsparende Weise mit einer entsprechend runden Lichteintrittsfläche.

Die aus dem Stand der Technik bekannten Lösungen erzeugen mittels Rechteck-Blenden oder Zylinderlinsen ein im Querschnitt rechteckiges Strahlenbündel, indem sie aus dem ursprünglich vollen, im Querschnitt runden Strahlenbüschel einen Teil ausklammern.

Ein Großteil des vorhandenen Lichts wird dadurch nicht genutzt. Bei einer erfindungsgemäßen Freiformfläcften-Linse hingegen wird immer das gesamte im Querschnitt runde Strahlenbüschel aufgenommen. Dieses kann mit einer viereckigen Lichteintrittsfläche (typisch für Platten oder Prismen) oder platzsparend mit einer runden Lichteintrittsfläche geschehen.

Die Freiformfläche, die die Oberflächentopographie einer solchen Linse beschreibt, ist durch ein Polynom höherer Ordnung (5. oder höher) darstellbar.

Die erfindungsgemäße Ausgestaltung einer beschriebenen Spaltlampe weist zusätzlich zu der Freiformflächen-Linse eine herkömmliche Spaltblende auf, obwohl auf die Letztere auch verzichtet werden könnte. Die Spaltblende hat nun nicht mehr die Funktion, den runden Querschnitt des Beleuchtungs-Strahlenbüschels in einen spaltförmigen, rechteckigen abzublenden, was mit hohem Lichtverlust verbunden wäre. Sie dient nur noch der Kontrastierung und der randscharfen Abbildung des schon durch die Freiformflächen-Linse erzeugten rechteckigen Querschnitts.

In Weiterentwicklung dieses Gedankens kann man bei einer anderen Ausführungsform der erfindungsgemäßen Spaltlampe mit entsprechend modifizierter Freiformfläche auf die Verwendung der Spaltblende überhaupt verzichten. Die Oberflächentopographie der Freiformfläche an der Linse muss dann so gestaltet sein, dass sie eine randscharfe, spaltförmige Abbildung des Querschnitts des Lichtbüschels am Patientenauge ermöglicht. Die spaltförmige Abbildung des Lichtbüschels am Patientenauge ergibt sich dann ausschließlich aus der Oberflächengestaltung der Freiformfläche der Linse.

Die Freiformflächen-Linse kann die Freiformfläche wahlweise auf der Licht-Eintritts-, der Licht-Austritts-Seite oder auf beiden Seiten aufweisen.

Eine erfindungsgemäße Ausgestaltungsvariante sieht vor, dass die Freiformflächen-Linse und auch die Spaltblende entlang der Achse des Beleuchtungs-Strahlengangs verschoben werden kann.

Eine weitere erfindungsgemäße Ausgestaltungsvariante ist auf möglichst vielfältige Anwendungsmöglichkeiten und Adaption an unterschiedliche Bedingungen ausgelegt. Hierfür ist es vorgesehen, dass die den rechteckigen Strahlenbüschel-Querschnitt erzeugende Freiformflächen-Linse gedreht und/oder aber auch entlang der optischen Achse der Spaltlampe verschoben werden kann. Durch die Drehung kann somit bei Bedarf nicht nur ein senkrecht stehender, sondern auch ein waagerecht oder schräg stehender "Licht-Spalt" erzeugt werden. Mit der Verschiebung entlang der optischen Achse wird bei Bedarf eine bessere Fokussierung auf eine optional nachgereihte Abbildungsoptik verfolgt. Es kann dadurch aber auch eine möglichst genaue Abstimmung des rechteckigen Strahlenbüschel-Querschnitts auf die rechteckige Öffnung der optional angeordneten Spaltblende erreicht werden, womit höherer Kontrast in der Randabbildung erzielt wird. Zusätzlich kann jedoch auch eine Verschiebbarkeit der Spaltblende vorgesehen sein.

Für die erfindungsgemäße Spaltlampe sollen sämtliche gängigen Leuchtmittel in Betracht kommen. Es kann sich hierbei z.B. um herkömmliche Glühbirnen, Lichtleiter oder LEDs handeln. Die Anordnung der Freiformflächen-Linse kann hierbei in unmittelbarer Nähe des Leuchtmittels oder in einer gewissen Entfernung erfolgen. Zwischen Leuchtmittel und Freiformfläche können auch Licht sammelnde optische Elemente angeordnet sein. Ersatzweise oder in Kombination kann auch ein Hohlspiegel eine Bündelung des vom Leuchtmittel austretenden Lichts vornehmen.

In einer weiteren erfindungsgemäßen Variante ist eine Freiformflächen-Linse direkt auf den Glaskörper des Leuchtmittels angebracht. Entsprechende Glaskörper können auch direkt einstückig gegossen werden. In diesem Fall kann zur besseren Fokussierbarkeit des Beleuchtungs-Strahlengangs eine Verschieb- und/oder Drehbarkeit des Leuchtmittels selbst vorgesehen sein.

Die beschriebene Beleuchtungsvorrichtung ist erfindungsgemäß auf zwei Arten mit einem Mikroskop kombinierbar: Der im Querschnitt modifizierte Beleuchtungs-Strahlengang wird mittels eines optischen Einspiegelungs-Elements in das Mikroskop eingebracht und durch das Hauptobjektiv geführt oder von außerhalb des Mikroskops als Schräg-Beleuchtung auf das Patientenauge geleitet. Letztere Ausgestaltungsvariante ist vorzugsweise so ausgestaltet, dass die Spalt-Beleuchtung bei Bedarf weggeschwenkt oder ausgeschaltet werden kann. In einer weiterhin bevorzugten Ausgestaltungsvariante ist die Schwenkvorrichtung hierfür so ausgestaltet, dass entlang eines Kreisbogens, der parallel zur Stereobasis des Mikroskops verläuft, geschwenkt werden kann. Diese Schwenkbewegung ermöglicht dem Fachmann eine Spalt-Beleuchtung mit unterschiedlichen Einfallswinkeln auf das Patientenauge und gewährleistet z.B. die Diagnostizierbarkeit von Trübungen in den transparenten Medien des Patientenauges.

Wenn die erfindungsgemäße Spalt-Beleuchtungseinrichtung mit einem Mikroskop, insbesondere einem Operationsmikroskop kombiniert ist, kann sie nach Belieben bei Bedarf zugeschaltet werden. Um jedoch eine weitere herkömmliche Beleuchtung des Mikroskops überflüssig zu machen, ist die erfindungsgemäße Spalt-Beleuchtungsvorrichtung so ausgestaltet, dass das den Licht-Spalt erzeugende optische Element mit Freiformfläche zu-schaltbar ist. Wenn es in den Beleuchtungs-Strahlengang eingebracht ist, dann sieht der Beobachter zu Diagnosezwecken am Patientenauge nur den Licht-Spalt, und wenn das optische Element nicht zugeschaltet ist, dann steht dem Beobachter eine Mikroskopbeleuchtung mit vollem Querschnitt zur Verfügung.

Anhand von Figuren wird die Erfindung symbolisch und beispielhaft näher erläutert.

Die Figuren werden zusammenhängend und übergreifend beschrieben. Gleiche Bezugszeichen bedeuten gleiche Bauteile, Bezugszeichen mit unter-schiedlichen Indices geben funktionsgleiche oder ähnliche Bauteile an.

Es zeigen dabei schematisch:
- Fig. 1 -: eine Spaltlampe nach dem Stand der Technik;
- Fig. 2 -: eine beispielhaft dargestellte Linse mit Freiformfläche;
- Fig. 3 -: die beispielhaft dargestellte Wirkungsweise einer Linse mit Freiformfläche;
- Fig. 3a -: einen runden Querschnitt eines Beleuchtungs-Strahlengangs;
- Fig. 3b -: einen rechteckigen Querschnitt eines Folge-Beleuchtungs-- Strahlengangs;
- Fig. 4 -: eine erfindungsgemäße Beleuchtungsvorrichtung als Spaltlampe mit einer Spaltblende und einer Abbildungsoptik;
- Fig. 5 -: eine Ausgestaltungsvariante einer erfindungsgemäßen Beleuchtungsvorrichtung als Spaltlampe;
- Fig. 6 -: eine erfindungsgemäße Beleuchtungsvorrichtung, die als Spaltlampe mit spaltförmigem Folge-Beleuchtungs-Strahlengang oder als Beleuchtung mit herkömmlichem Beleuchtungs-Strahlengang mit einem Mikroskop kombiniert ist;
- Fig. 7 -: einen erfindungsgemäßen Aufbau eines Mikroskops mit einer ein-schwenkbaren Beleuchtungsvorrichtung als Spaltlampe und
- Fig. 8 -: ein Leuchtmittel mit einer Freiformflächen-Linse.

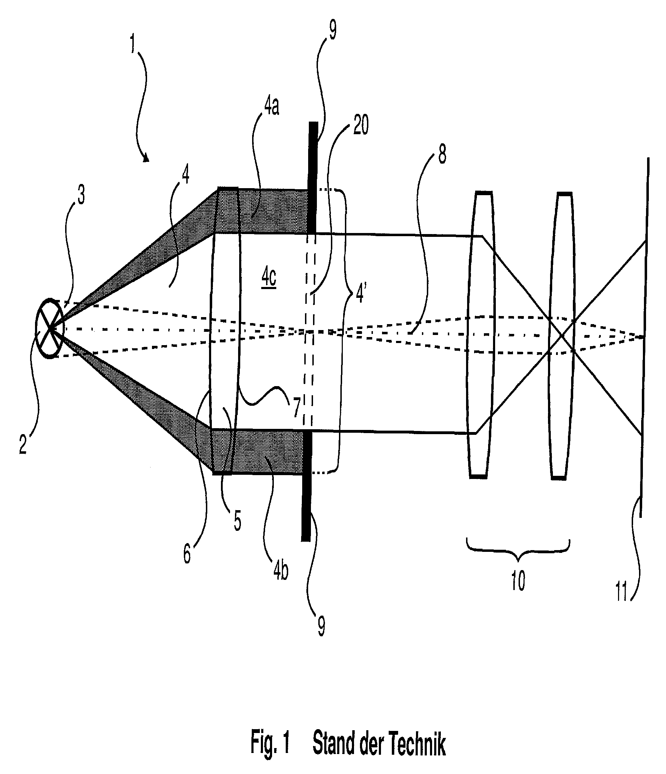

Fig. 1 zeigt eine Längsschnitt-Darstellung einer Beleuchtungsvorrichtung 1, die gemäß Stand der Technik als Spaltlampe eingesetzt wird. Hierbei erzeugt ein Leuchtmittel 2 einen Abbildungs-Strahlengang 3 und einen Beleuchtungs-Strahlengang 4, die entlang einer optischen Achse 8 verlaufen. Diese beiden Strahlengänge treffen auf ein optisches Element 5 (in der Regel eine Sammellinse) auf, das eine Lichteintritts-Fläche 6 und eine Lichtaustritts-Fläche 7 aufweist. Das optische Element 5 bündelt den Beleuchtungs-Strahlengang 4 auf eine Spaltblende 9, die eine spaltförmige Öffnung 20 hat. Dadurch kann nur noch das der Spaltblenden-Öffnung 20 entsprechende Lichtbüschel des Beleuchtungs-Strahlengangs 4 die Spaltblende 9 passieren. Der Folge-Beleuchtungs-Strahlengang 4' setzt sich somit aus einem tatsächlich verwerteten Teil 4c des Beleuchtungs-Strahlengangs 4, und - gemäß dieser Schnittdarstellung - zwei nicht verwerteten Teilen 4a und 4b, die von der Spaltblende 9 absorbiert und in Wärme umgewandelt werden, zusammen. Der so übrig gebliebene, spaltförmige Lichtstrahl wird dann durch eine Abbildungsoptik 10 auf der Beleuchtungsebene 11 abgebildet, die bei einer Anwendung als Spaltlampe in der Regel ein Patientenauge ist. Diese herkömmliche Spaltlampen-Technik ist mit schlechter Licht-Ausnutzung und hoher Wärmeentwicklung verbunden.

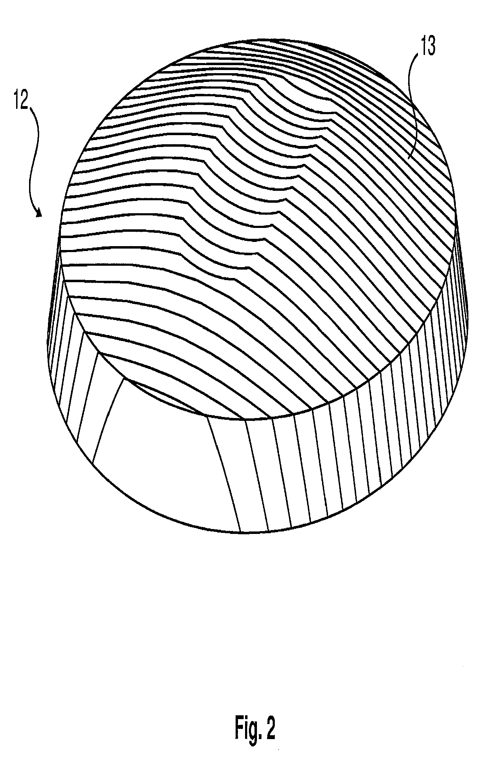

Fig. 2 zeigt beispielhaft eine Freiformflächen-Linse 12, die eine Freiformfläche 13 aufweist. Letztere ist als eine unregelmäßige, von einer Sphäre abweichende Fläche dargestellt.

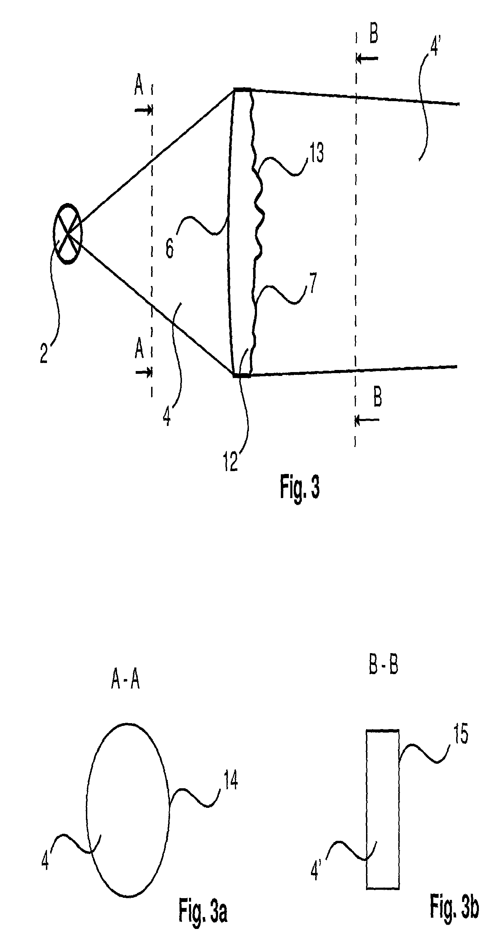

Fig. 3 zeigt die Wirkungsweise einer Freiformflächen-Linse 12 mit einer Freiformfläche 13, die einen Beleuchtungs-Strahlengang 4 von einem Leucht-mittel 2 aufnimmt. Die Lichteintritts-Fläche 6 ist herkömmlich sphärisch ausgebildet, die Lichtaustritts-Fläche 7 ist als Freiformfläche 13 ausgestaltet. Der Beleuchtungs-Strahlengang 4 weist einen runden Querschnitt 14 (Schnittdarstellung A-A in Fig. 3a) und der Folge-Befeuchtungs-Strahlengang 4' weist einen rechteckigen bzw. spaltförmigen Querschnitt 15 (Schnittdarstellung B-B in Fig. 3b) auf.

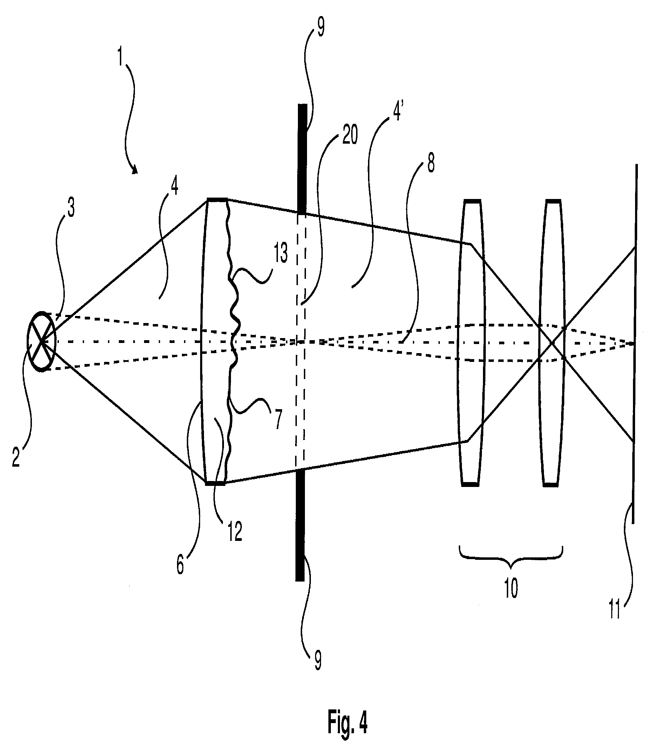

Fig. 4 zeigt den erfindungsgemäßen Einsatz einer Freiformflächen-Linse 12, deren Lichtaustritts-Fläche 7 als Freiformfläche 13 ausgestaltet ist, in einer Beleuchtungsvorrichtung bzw. Spaltlampe 1. Obwohl die Anordnung einer Spaltblende 9 nun nicht mehr erforderlich ist, weil der Folge-Beleuchtungs-Strahlengang 4' schon einen spaltförmigen Querschnitt aufweist, ist zur besseren Konturierung des spaltförmigen Strahls eine solche vorgesehen.

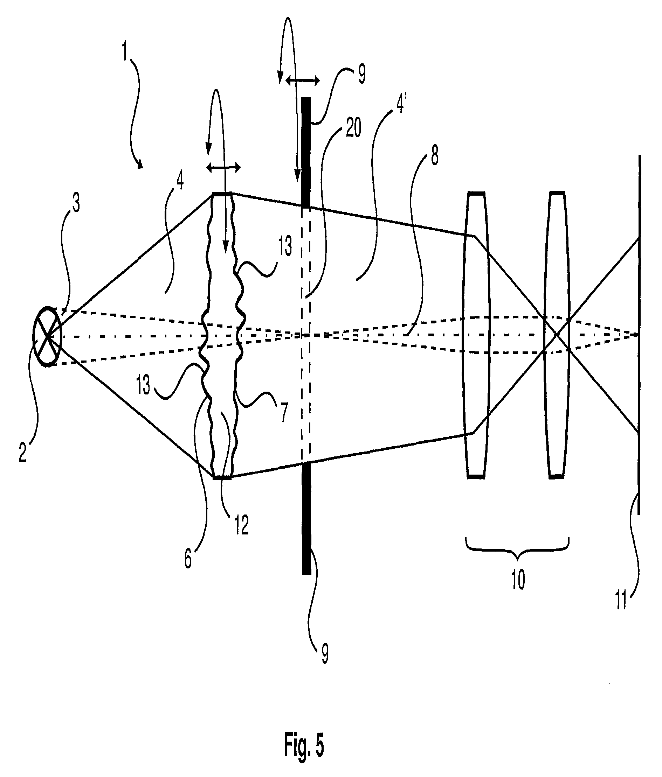

Fig. 5 zeigt eine weitere erfindungsgemäße Ausgestaltungsvariante einer Beleuchtungsvorrichtung 1 mit einer Freiformflächen-Linse 12, die sowohl an der Lichteintritts-Fläche 6, als auch an der Lichtaustritts-Fläche 7 eine Freiformfläche 13 aufweist. Des Weiteren sind sowohl die Freiformflächen-Linse 12, als auch die Spaltblende 9 entlang der optischen Achse 8 verschiebbar und um sie drehbar angeordnet. Dadurch kann Einfluss auf Kontur, Position und Lichtstärke des auf der Beleuchtungsebene (Patientenauge) 11 abgebildeten Licht-Spalts genommen werden.

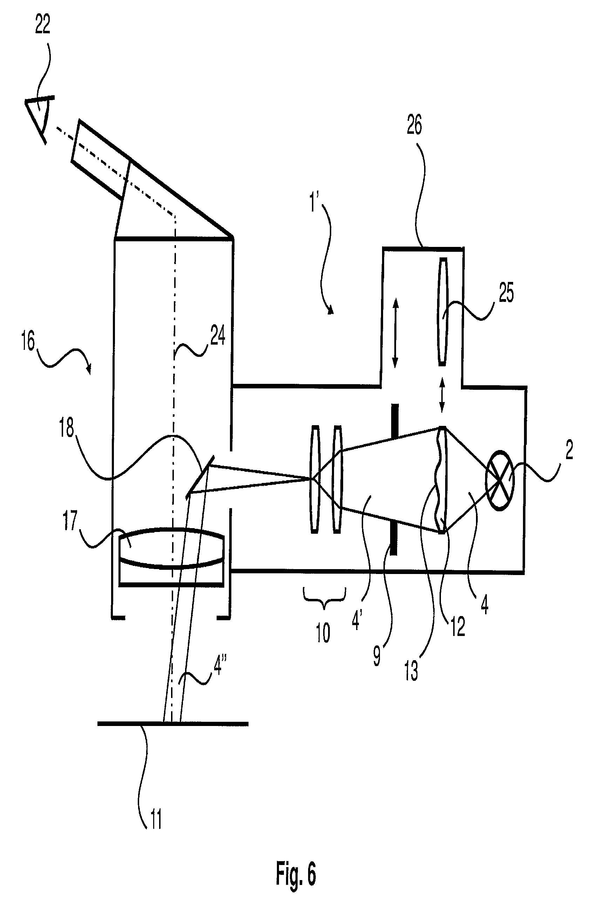

Fig. 6 zeigt eine erfindungsgemäße Kombination einer neu vorgestellten Beleuchtungsvorrichtung bzw. Spaltlampe 1 mit einem Mikroskop 16. Die Spaltlampe ist am Mikroskop 16 so angeordnet, dass der spaltförmige Strahl von einem Einspiegelungselement 18 durch ein Hauptobjektiv 17 auf die Beleuchtungsebene bzw. das Patientenauge 11 gelenkt wird. Das Einspiegelungselement 18 kann so ausgerichtet sein, dass der Folge-Beleuchtungs-Strahlengang 4" das Sehfeld-Zentrum des Mikroskops beleuchtet, das um eine optische Achse 24 des Mikroskops 16 liegt. Des Weiteren weist diese Ausgestaltungsvariante eine Austausch-Vorrichtung 26 auf, die es ermöglicht, dass die Spaltblende 9 verschoben oder verschwenkt werden kann und auch die Freiformflächen-Linse 12 auf ähnliche Weise mit einer herkömmlich geformten Austausch-Linse 25 ausgetauscht werden kann. Dadurch ist die Beleuchtungsvorrichtung 1' wahlweise als herkömmliche Mikroskopbeleuchtung oder als Spaltlampe verwendbar.

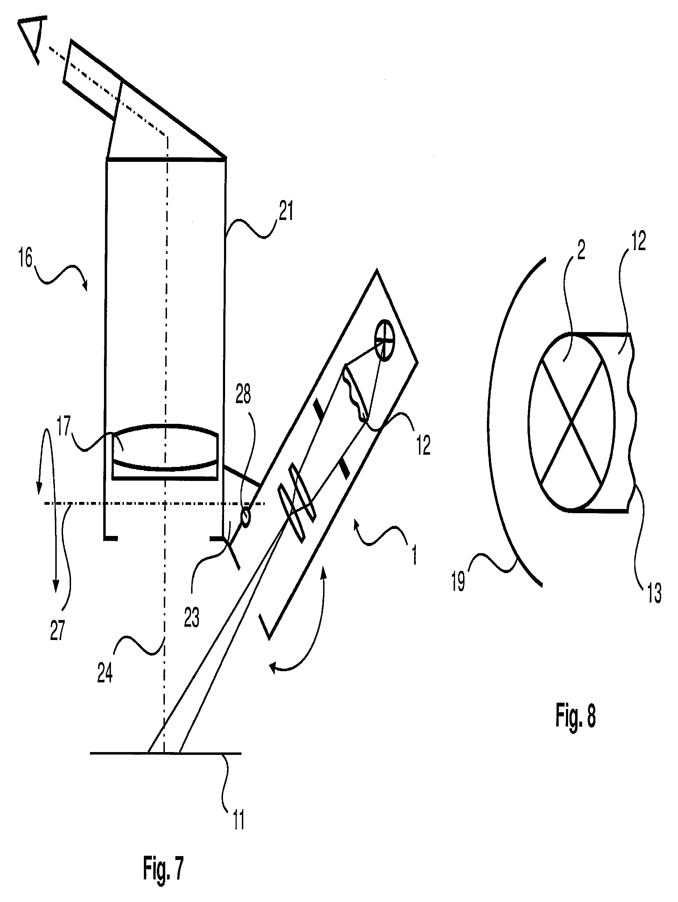

Fig. 7 zeigt eine Beleuchtungsvorrichtung bzw. Spaltlampe 1, die mittels einer Schwenkvorrichtung 23 bei Bedarf ein- oder ausgeschwenkt werden kann. Die Schwenkvorrichtung 23 stellt zwei unterschiedliche Schwenkrichtungen zur Verfügung. Eine Schwenkbewegung verläuft um das Gelenk 28 und dient der Zu- oder Abschaltung der Spalt-Beleuchtung. Die zweite Schwenkrichtung verläuft um die Schwenkachse 27, also parallel zu der Stereobasis des Mikroskops 16. Letztere Schwenkbewegung ist für Diagnosezwecke wichtig und dem Fachmann bekannt.

Fig. 8 zeigt ein Leuchtmittel 2, das direkt mit einer Freiformflächen-Linse 12 ausgestattet ist. Eine Kombination beider könnte auch einstückig ausgebildet sein. Ein Hohlspiegel 19 sorgt dafür, dass ein Teil des nach links austretenden Lichts auch der Freiformflächen-Linse 12 zugeführt wird.

## Patentansprüche

1. Beleuchtungsvorrichtung (1) mit einem Leuchtmittel (2) und einem Beleuchtungs-Strahlengang (4) mit rundem Querschnitt (14), sowie mindestens einem auf einer optischen Achse (8) angeordneten Licht brechenden optischen Element (5) mit einer Licht-Eintrittsfläche (6) und einer Licht-Austrittsfläche (7), **dadurch gekennzeichnet, dass** das optische Element (5) an wenigstens einer der beiden Flächen (6, 7) mit einer Freiformfläche (13) versehen ist, die so ausgeformt ist, dass im Betriebszustand alles Licht aus dem Beleuchtungs-Strahlengang (4) zu einem Folge-Beleuchtungs-Strahlengang (4') mit drei-, vier-, vieleckigem, halbrundem oder sicheltörmigem Querschnitt (15) bündelbar ist.

2. Beleuchtungsvorrichtung (1) nach Anspruch 1, **dadurch gekennzeichnet, dass** das optische Element (5) eine Linse, eine Platte oder ein Prisma ist.

3. Beleuchtungsvorrichtung (1) nach einem der vorhergehenden Ansprüche, **dadurch gekennzeichnet, dass** das optische Element (5) sowohl an der Licht-Eintrittsfläche (6) als auch an der Licht-Austrittsfläche (7) eine Freiformfläche (13) aufweist.

4. Beleuchtungsvorrichtung (1) nach einem der vorhergehenden Ansprüche, **dadurch gekennzeichnet, dass** der Querschnitt (15) des Folge-Beleuchtungs-Strahlengangs (4') einen spaltförmigen Querschnitt (15) aufweist und die Beleuchtungsvorrichtung (1) als Spaltlampe einsetzbar ist.

5. Beleuchtungsvorrichtung (1) nach Anspruch 4, **dadurch gekennzeichnet, dass** der im Querschnitt (15) spaltförmige Beleuchtungs-Strahlengang (4') auf eine entsprechend spaltförmige Öffnung (20) einer nachgeordneten Spaltblende (9) fokussierbar ist.

6. Beleuchtungsvorrichtung (1) nach Anspruch 5, **dadurch gekennzeichnet, dass** die Spaltblende (9) entlang der optischen Achse (8) verschiebbar und /oder um die optische Achse (8) drehbar ausgestaltet ist.

7. Beleuchtungsvorrichtung (1) nach einem der vorhergehenden Ansprüche, **dadurch gekennzeichnet, dass** das optische Element (5) entlang der optischen Achse (8) verschiebbar und/oder um die optische Achse (8) drehbar ausgestaltet ist.

8. Beleuchtungsvorrichtung (1) nach einem der vorhergehenden Ansprüche, **dadurch gekennzeichnet, dass** dem optischen Element (5) auf der opti-schen Achse (8) eine Abbildungsoptik (10) nachgeordnet ist.

9. Beleuchtungsvorrichtung (1) nach einem der vorhergehenden Ansprüche, **dadurch gekennzeichnet, dass** das optische Element (5) unmittelbar am Leuchtmittel (2) angeordnet ist und das Leuchtmittel (2) entlang der optischen Achse (8) verschiebbar und/oder um die optische Achse (8) drehbar ausgestaltet ist.

10. Beleuchtungsvorrichtung (1) nach Anspruch 9, **dadurch gekennzeichnet, dass** das optische Element (5) und der Glaskörper des Leuchtmittels (2) einstückig ausgestaltet sind.

11. Beleuchtungsvorrichtung (1) nach einem der vorhergehenden Ansprüche, **dadurch gekennzeichnet, dass** der Beleuchtungs-Strahlengang (4'), umgelenkt durch ein Einspiegelungselement (18) eines Mikroskops (16), im Betriebszustand die Beleuchtungsebene (11) durch ein Hauptobjektiv (17) des Mikroskops (16) beleuchtet.

12. Beleuchtungsvorrichtung (1) nach einem der Ansprüche 1-10, **dadurch gekennzeichnet, dass** der Beleuchtungs-Strahlengang (4') für die Beleuchtungsebene (11) außerhalb des Mikroskops (16) angeordnet ist und die Beleuchtungsvorrichtung (1) schwenkbar am Mikroskopkörper (21) befestigt ist.

13. Beleuchtungsvorrichtung (1) nach Anspruch 11 oder 12, **dadurch gekennzeichnet, dass** im Betriebszustand des Mikroskops (16) das optische Element (5) mit Freiformfläche (13) bei Bedarf zuschaltbar ist, sodass bei Bedarf ein Spaltlampen-Diagnosemodus oder ein normaler Beleuchtungsmodus schaltbar ist.

14. Verwendung mindestens eines optischen Elements (5) mit Freiformflächen (13), die durch ein Polynom 5. oder höherer Ordnung beschreibbar sind, in Beleuchtungs-Strahlengängen (4) von Beleuchtungsvorrichtungen (1), insbesondere Spaltlampen und Mikroskopbeleuchtungen.
